# EUROPÄISCHE PATENTSCHRIFT

(11) **EP 0 345 573 B1**
(45) Veröffentlichungstag und Bekanntmachung des Hinweises auf die Patenterteilung: **21.04.1993**
(21) Anmeldenummer: 89109593.7
(22) Anmeldetag: 27.05.1989
(51) Int. Cl.: C07C 233/31, C07D 231/12, C08F 20/58, G03C 1/00

(54) **2-[(Meth)Acrylamidomethyl]-1,3-diketone**
2-[(Meth)acrylamidomethyl]-1,3-diketones
2-[(Méth)Acrylamidométhyl]-1,3-dicétone

(30) Priorität: 08.06.1988 DE 3819455
(43) Veröffentlichungstag der Anmeldung: 13.12.1989
(73) Patentinhaber: BASF Aktiengesellschaft, 67063 Ludwigshafen (DE)
(72) Erfinder: Nick, Bernhard, Dr., D-6700 Ludwigshafen (DE); Bott, Kaspar, Dr., D-6800 Mannheim 1 (DE); Schulz, Guenther, Dr., D-6702 Bad Duerkheim (DE)

(56) Entgegenhaltungen:
- EP-A- 0 188 037
- US-A- 3 864 311

## Beschreibung

Die vorliegende Erfindung betrifft neue 2-[(Meth)Acrylamidomethyl]-1,3-diketone.

Chelatisierende Verbindungen, welche mit Metallkationen 5- oder 6-gliedrige Ringe bilden, sind allgemein bekannt. Ein charakteristisches Beispiel hierfür ist das Acetylaceton. Verbindungen dieser Art bieten viele Anwendungsmöglichkeiten, z.B. bei der Isolierung oder Fixierung von Metallen oder bei der Herstellung gut haftender Schutzschichten auf Metalloberflächen. Besonders groß ist das Interesse an chelatisierenden Verbindungen, welche eine zur radikalischen Polymerisation befähigte, olefinische Doppelbindung aufweisen. Verbindungen dieser Art können nämlich zur Herstellung von Polymerisaten mit außergewöhnlichen Eigenschaften und zahlreichen Anwendungsmöglichkeiten dienen.

Verbindungen, welche eine chelatisierende 1,3-Diketon-Gruppe und einen zur radikalischen Polymerisation befähigten, olefinisch ungesättigten Rest enthalten und welche daher auch kurz als chelatisierende Monomere bezeichnet werden, sind z.B. aus der EP-A-188 037 bekannt. In diesen bekannten Verbindungen ist die 1,3-Diketon-Gruppe über eine oder zwei Esterfunktionen mit dem (Meth)Acrylsäurerest verbunden. Charakteristische Beispiele hierfür sind

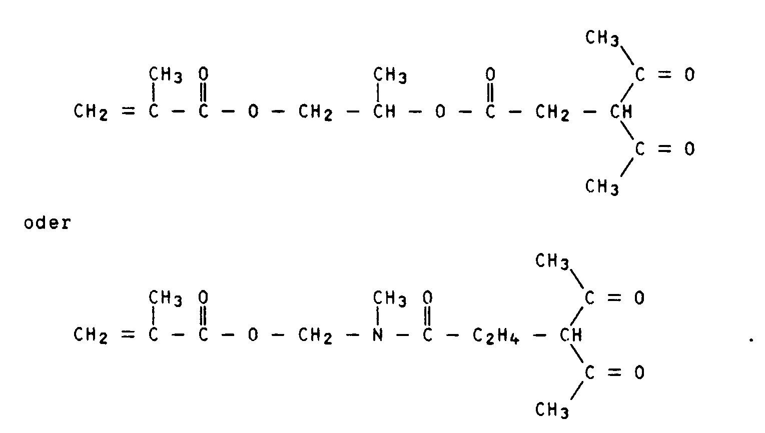

Diese bekannten chelatisierenden Monomeren haben jedoch den Nachteil, daß die darin enthaltenen Esterfunktionen leicht verseifbar sind. Deswegen kommt es in Gegenwart von Wasser, wäßrig-alkalischen Lösungen, Ammoniak oder Aminen leicht zu einer Zersetzung dieser Verbindungen. Sie kommen daher nur für Anwendungszwecke in Betracht, bei denen die Gefahr der Zersetzung und damit verbunden des Verlusts der charakteristischen Eigenschaften nicht besteht. Überdies sind bei der Herstellung dieser Verbindungen mehrere Syntheseschritte für die Verknüpfung der 1,3-Diketongruppe mit dem (Meth)Acryloylrest erforderlich, was die Attraktivität dieser Verbindungen noch weiter vermindert.

Aufgabe der vorliegenden Erfindung war es, stabile und einfach herstellbare Verbindungen mit einer 1,3-Diketongruppe und einen radikalisch polymerisierbare, insbesondere photopolymerisierbare, olefinische Doppelbindung enthaltenden Rest aufzuzeigen, welche die Nachteile des Standes der Technik nicht mehr aufweisen.

Es wurde nun überraschend gefunden, daß diese Aufgabe durch die neuen 2-[(Meth)Acrylamidomethyl]-1,3-diketone der nachfolgend näher bezeichneten Art gelöst wird.

Gegenstand der Erfindung sind dementsprechend 2-[(Meth)Acrylamidomethyl]-1,3-diketone der allgemeinen Formel (I) 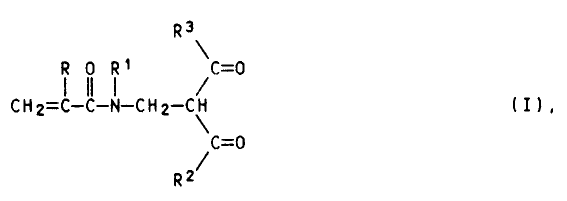
worin die Reste R, R¹, R² und R³, unabhängig voneinander, die folgende Bedeutung haben
- R:: ein Wasserstoffatom oder eine Methylgruppe, vorzugsweise ein Wasserstoffatom;
- R¹:: ein Wasserstoffatom, eine Methyl- oder eine Ethylgruppe, vorzugsweise ein Wasserstoffatom;
- R², R³:: eine Alkylgruppe mit 1 bis 6 C-Atomen oder eine unsubstituierte oder, beispielsweise durch Alkyl, Aryl oder Halogen, substituierte Arylgruppe mit 6 bis 20 C-Atomen, wobei R² und R³ gleich oder voneinander verschieden sein können.

Beispiele für die Reste R² und R³ in der allgemeinen Formel (I) sind: Methyl, Ethyl, n-Propyl, i-Propyl, n-Butyl, sec.-Butyl, tert.-Butyl, Pent-1-, -2- oder -3-yl sowie Hex-1-, -2- oder -3-yl; weiterhin Phenyl, 2-, 3-und 4-Methyl-phenyl, 2,4-Dimethylphenyl, 4-tert.-Butyl-, 4-Chlor- und 4-Brom-phenyl, 4-Phenyl-phen-1-yl (Biphenylyl), 4-(4′-Phenyl-phen-1′-yl)-phen-1-yl (Triphenylyl), 1- und 2-Naphthyl, Phenanthren-7-yl, Anthracen-1-yl, Fluoren-2-yl und Perylen-3-yl. Hierbei sind Methyl, Ethyl, n-Propyl und Phenyl erfindungsgemäß bevorzugt, wobei Methyl und Phenyl ganz besonders bevorzugt sind.

Beispiele bevorzugter erfindungsgemäßer 2-[(Meth)Acrylamidomethyl]-1,3-diketone der allgemeinen Formel (I) sind

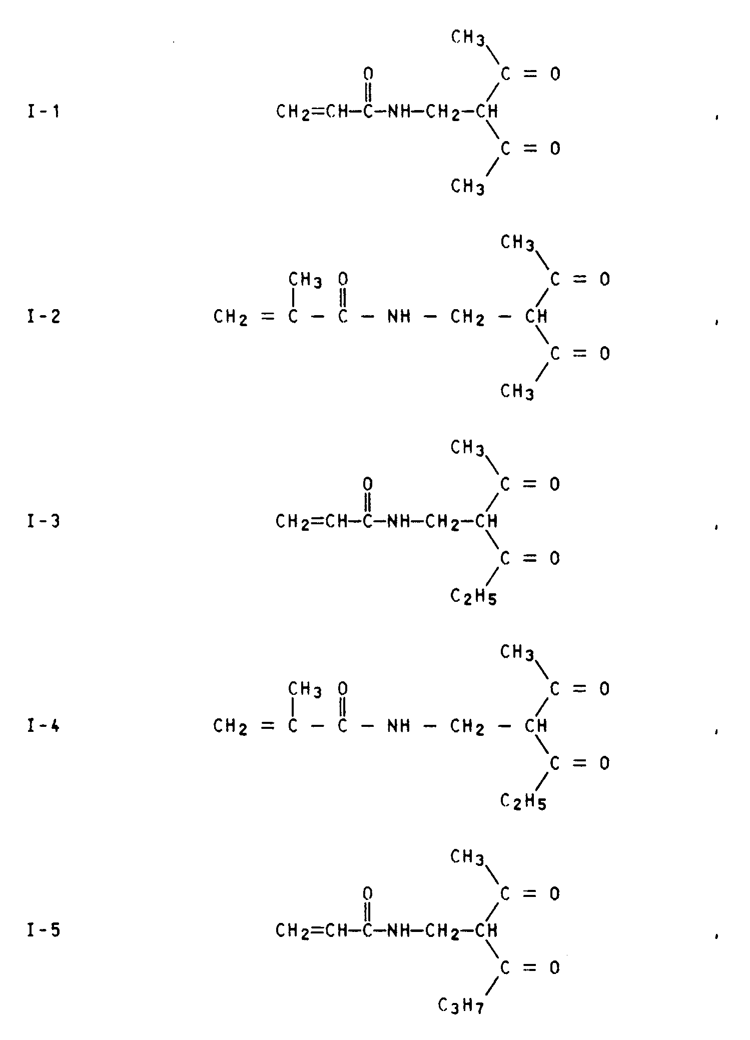

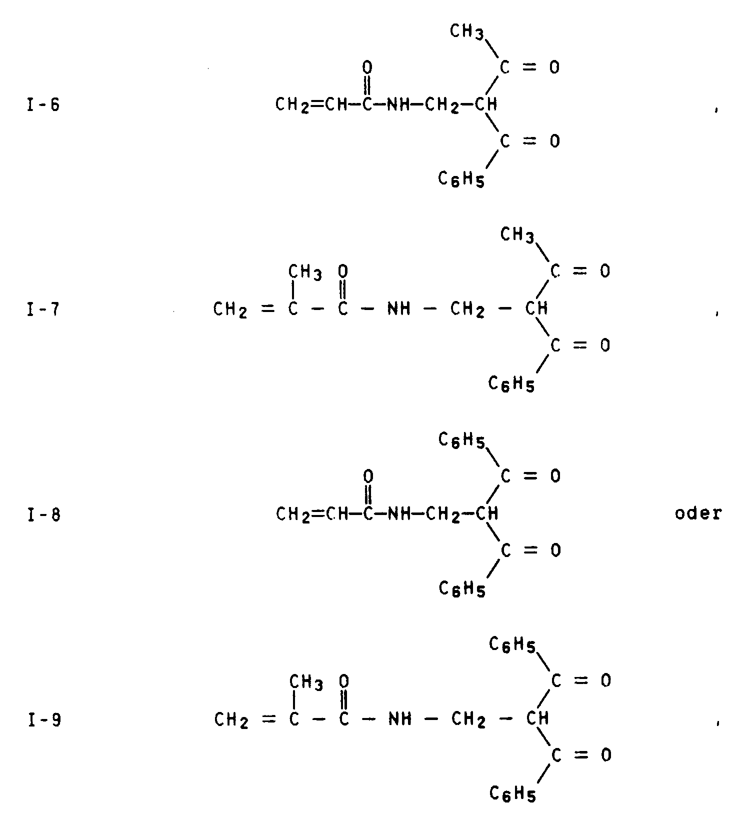

Von diesen sind die 2-[(Meth)Acrylamidomethyl]-1,3-diketone der Formeln I-1 [3-(Acrylamidomethyl)-2,4-pentandion], I-6 [1-Phenyl-2-(acrylamidomethyl)-1,3-butandion] und I-8 [1,3-Diphenyl-2-(acrylamidomethyl)-1,3-propandion] ganz besonders bevorzugt.

Die Herstellung der erfindungsgemäßen 2-[(Meth)Acrylamidomethyl]-1,3-diketone der allgemeinen Formel (I) kann nach den üblichen und bekannten Methoden der präparativen organischen Chemie erfolgen. Von Vorteil ist es indes, die erfindungsgemäßen 2-[(Meth)Acrylamidomethyl]-1,3-diketone der allgemeinen Formel (I) nach erfindungsgemäßer Verfahrenweise durch Amidomethylierung von 1,3-Diketonen in Gegenwart starker Säuren herzustellen.

Dieses erfindungsgemäße Verfahren geht aus von einem 1,3-Diketon der allgemeinen Formel (II) 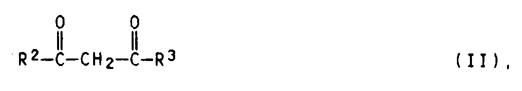
worin die Reste R² und R³ gleich oder voneinander verschieden sein können und die vorstehende, im Zusammenhang mit der allgemeinen Formel (I) angegebene Bedeutung haben. Beispiele für die Reste R² und R³ in der allgemeinen Formel (II) sind ebenfalls die für die Reste R² und R³ in der allgemeinen Formel (I) genannten Beispiele. Bevorzugte Reste R² und R³ sind Methyl, Ethyl, n-Propyl und Phenyl, insbesondere Methyl und Phenyl.

Beispiele für 1,3-Diketone der allgemeinen Formel (II), welche bei dem erfindungsgemäßen Verfahren ganz besonders bevorzugt verwendet werden, sind 2,4-Pentandion (II-1), Benzoylaceton (II-2) und 1,3-Diphenyl-1,3-propandion (II-3).

In erfindungsgemäßer Verfahrensweise werden die 1,3-Diketone der allgemeinen Formel (II) in einer ausreichenden Menge einer starken Säure als Reaktionsmedium bei -5 bis +10°C, insbesondere bei etwa 0°C, gelöst oder dispergiert. Eine geeignete Menge an Säure ist im allgemeinen etwa das 2-bis 20-fache des Gewichts des 1,3-Diketons der allgemeinen Formel (II). Geeignete starke Säuren sind unter anderem konzentrierte Schwefelsäure, konzentrierte Phosphorsäure oder konzentrierte Trifluormethansulfonsäure, von denen erstere besonders günstig ist.

Zu dieser Lösung oder Dispersion des 1,3-Diketons der allgemeinen Formel (II) fügt man dann die äquimolare Menge eines N-Methylol-(meth)acrylamides der allgemeinen Formel (III) hinzu, 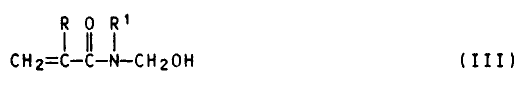
worin der Rest R ein Wasserstoffatom oder eine Methylgruppe und, unabhängig hiervon, der Rest R¹ ein Wasserstoffatom, eine Methyl- oder eine Ethylgruppe bedeuten. Erfindungsgemäß bevorzugt sind hierbei N-Methylol-(meth)acrylamide der allgemeinen Formel (III), worin R¹ ein Wasserstoffatom bedeutet. Ganz besonders bevorzugt ist das N-Methylol-acrylamid. Die N-Methylol-(meth)acrylamide der allgemeinen Formel (III) können bekanntermaßen in einfacher Weise aus Formaldehyd und dem entsprechenden (Meth)Acrylamid hergestellt werden.

Erfindungsgemäß ist es von Vorteil, dem resultierenden Reaktionsgemisch einen üblichen und bekannten thermischen Polymerisationsinhibitor zuzusetzen. Geeignete Polymerisationsinhibitoren sind z.B. Trisnonylphenylphosphit, 2,6-Di-tert.-butyl-p-kresol, Hydrochinonmonomethylether oder polymerisiertes Trimethyldihydrochinon, von denen Hydrochinonmonomethylether besonders vorteilhaft ist. Die Polymerisationsinhibitoren werden dem Reaktionsgemisch in aller Regel in Mengen von 0,001 bis 5 Gew.%, bezogen auf das N-Methylol-(meth)acrylamid der allgemeinen Formel (III), zugegeben.

Das resultierende Reaktionsgemisch aus dem 1,3-Diketon der allgemeinen Formel (II), dem N-Methylol-(meth)acrylamid der allgemeinen Formel (III), der starken Säure sowie gegebenenfalls dem Polymerisationsinhibitor wird dann bei 15 bis 40°C, vorzugsweise bei 15 bis 30°C und insbesondere bei Temperaturen im Bereich von 20 bis 25°C, für eine Dauer von 1 bis 48 Stunden, vorzugsweise 2 bis 40 Stunden und insbesondere 2,5 bis 20 Stunden, gerührt. In dieser Zeit läuft die Kondensationsreaktion zwischen dem 1,3-Diketon der allgemeinen Formel (II) und dem N-Methylol-(meth)acrylamid der allgemeinen Formel (III) zu den erfindungsgemäßen 2-[(Meth)Acrylamidomethyl]-1,3-diketonen der allgemeinen Formel (I) ab.

Hiernach gießt man das umgesetzte Reaktionsgemisch auf einen mengenmäßig hohen Überschuß an, vorzugsweise gemahlenem, Eis, wodurch das erfindungsgemäße 2-[(Meth)Acrylamidomethyl]-1,3-diketon der allgemeinen Formel (I) ausgefällt wird. Das ausgefällte Produkt sowie in der wäßrigen Phase gegebenenfalls noch vorhandenes Produkt werden dann mit einem organischen Lösungsmittel, wie z.B. Toluol, Xylol oder halogenierten Kohlenwasserstoffen, wie Dichlormethan, extrahiert und nach dem Abdampfen des Lösungsmittels aus geeigneten Lösungsmitteln oder Lösungsmittelgemischen umkristallisiert.

Das erfindungsgemäße Verfahren kann in beliebigen Anlagen oder Apparaturen aus Glas oder Metallen ausgeführt werden, wie sie auf dem Gebiet der präparativen organischen Chemie üblich und gebräuchlich sind.

Die erfindungsgemäßen 2-[(Meth)Acrylamidomethyl]-1,3-diketone der allgemeinen Formel (I) können aufgrund ihrer besonderen vorteilhaften Eigenschaften anstelle der aus der EP-A-188 037 bekannten chelatisierenden Monomeren mit besonderem Vorteil für alle in dieser Druckschrift beschriebenen Anwendungszwecke verwendet werden. Hierbei sind sie den bekannten chelatisierenden Monomeren unter anderem schon deshalb überlegen, weil sie erheblich hydrolysestabiler als diese sind. Überdies können die erfindungsgemäßen 2-[(Meth)Acrylamidomethyl]-1,3-diketone der allgemeinen Formel (I) in einfacherer Weise als die bekannten chelatisierenden Monomeren hergestellt werden, weswegen sie für die industrielle Anwendung von besonderem Interesse sind. Die erfindungsgemäßen 2-[(Meth)Acrylamidomethyl]-1,3-diketone der allgemeinen Formel (I) können mit Vorteil als wertvolle synthetische Zwischenprodukte für die Herstellung polymerisierbarer Metallchelat-Komplexe oder von Polymerisaten, welche zur Metallchelat-Komplexbildung befähigt sind, verwendet werden. Oder sie können als photopolymerisierbare Monomere in lichtempfindlichen Aufzeichnungsmaterialien angewandt werden. Diese lichtempfindlichen Aufzeichnungsmaterialien, welche üblicherweise der Herstellung von Reliefplatten, Hochdruck-, Flexodruck-, Tiefdruck- oder Offsetdruckformen oder auch von bildmäßig strukturierten Resistschichten dienen, weisen wegen ihres Gehaltes an 2-[(Meth)Acrylamidomethyl]-1,3-diketonen der allgemeinen Formel (I) besondere unerwartete Vorteile auf. Sehr günstig ist auch die Verwendung der erfindungsgemäßen 2-[(Meth)Acrylamidomethyl]-1,3-diketone der allgemeinen Formel (I) als Zwischenprodukte für die Herstellung polymerisierbarer heterocyclischer Verbindungen. Hierzu werden die erfindungsgemäßen 2-[(Meth)Acrylamidomethyl]-1,3-diketone der allgemeinen Formel (I) z.B. mit Hydroxylamin, Hydroxylaminderivaten, Hydrazin oder Hydrazinderivaten in einfacher Weise mit hohen Ausbeuten zu (Meth)Acrylamidomethyl-Reste enthaltenden Isoxazolen oder Pyrazolen umgesetzt. Hierbei besteht nicht die Gefahr, daß die Verknüpfung zwischen der 1,3-Diketon-Gruppe oder der heterocyclischen Gruppe und dem zur radikalischen Polymerisation befähigten (Meth)Acryloyl-Rest durch unerwünschte Nebenreaktionen gespalten wird.

### Beispiele

### Beispiel 1

### Herstellung und Charakterisierung von 3-(Acrylamidomethyl)-2,4-pentandion

In 500 ml konzentrierte Schwefelsäure wurden bei 0°C der Reihe nach 1,0 g Hydrochinonmonomethylether, 64,5 g 2,4-Pentandion und 64,8 g N-Methylolacrylamid eingetragen. Das Reaktionsgemisch wurde anschließend 4 Stunden lang bei 20°C gerührt, wonach es ausreagiert war und auf 2 kg gemahlenes Eis gegossen wurde. Das hierbei ausgefällte 3-(Acrylamidomethyl)-2,4-pentandion wurde in Dichlormethan gelöst und die resultierende Lösung von der wäßrigen Phase abgetrennt. Anschließend wurde das Lösungsmittel verdampft und das resultierende Produkt aus Toluol umkristallisiert.

Es wurden 60,3 g rohes 3-(Acrylamidomethyl)-2,4-pentandion erhalten, was einer Ausbeute von 51 Gew.%, bezogen auf die Ausgangsprodukte, entspricht. Das erhaltene Produkt hatte einen Schmelzpunkt von 108 bis 110°C. Die elementare Zusammensetzung des Produktes in Gew.% wurde mittels der üblichen und bekannten chemischen Analysenmethoden bestimmt:

| | | | | |
|---|---|---|---|---|
| Elementaranalyse: | C 59,0 | H 7,0 | O 26,3 | N 7,7 |
| Theorie: | C 59,0 | H 7,16 | O 26,2 | N 7,65 |

Die ¹H-NMR- und IR-spektroskopischen Messungen bestätigten das Vorliegen der gewünschten Verbindung.

### Beispiel 2

### Herstellung und Charakterisierung von 1-Phenyl-2-(acrylamidomethyl)-1,3-butandion

Analog zu der in Beispiel 1 angegebenen Vorschrift wurde 1-Phenyl-2-(acrylamidomethyl)-1,3-butandion aus 100 g Benzoylaceton und 64,8 g N-Methylol-acrylamid hergestellt.

Nach der Umkristallisation des Produktes in einem Gemisch aus 3 Vol.-Teilen Cyclohexan und 1 Vol.-Teil Ethanol resultierten 105 g 1-Phenyl-2-(acrylamidomethyl)-1,3-butandion, entsprechend einer Reinausbeute von 70 Gew.%. Das Produkt hatte einen Schmelzpunkt von 125 bis 126°C.

| | | | | |
|---|---|---|---|---|
| Elementaranalyse: | C 68,7 | H 6,0 | O 19,7 | N 5,7 |
| Theorie: | C 68,56 | H 6,16 | O 19,57 | N 5,71 |

Die ¹H-NMR- und IR-spektroskopischen Messungen bestätigten das Vorliegen der gewünschten Verbindung.

### Beispiel 3

### Herstellung und Charakterisierung von 1,3-Diphenyl-2-(acrylamidomethyl)-1,3-propandion

Analog zu der in Beispiel 1 angegebenen Vorschrift wurde 1,3-Diphenyl-2-(acrylamidomethyl)-1,3-propandion aus 143 g 1,3-Diphenyl-1,3-propandion und 64,8 g N-Methylol-acrylamid hergestellt.

Nach dem Umkristallisieren des Produkts in Methanol wurden 127 g 1,3-Diphenyl-2-(acrylamidomethyl)-1,3-propandion erhalten, was einer Reinausbeute von 65 Gew.% entspricht. Das Produkt hatte einen Schmelzpunkt von 146 bis 148°C.

| | | | | |
|---|---|---|---|---|
| Elementaranalyse: | C 74,1 | H 5,6 | O 15,8 | N 4,5 |
| Theorie: | C 74,25 | H 5,58 | O 15,62 | N 4,56 |

Die ¹H-NMR- und IR-spektroskopischen Messungen bestätigten das Vorliegen der gewünschten Verbindung.

### Beispiele 4 bis 7

Verwendung erfindungsgemäßer 2-[(Meth)Acrylamidomethyl]-1,3-diketone als Zwischenprodukte für die Herstellung radikalisch polymerisierbarer Heterocyclen

### Beispiel 4

### 3-Phenyl-4-(acrylamidomethyl)-5-methyl-isoxazol

Eine Mischung aus 34,6 g des nach Beispiel 2 hergestellten 1-Phenyl-2-(acrylamidomethyl)-1,3-butandions, 9,84 g Hydroxylammoniumchlorid, 11,6 g Natriumacetat, 180 ml Essigsäure und 180 ml Wasser wurde 5 Minuten lang am Rückfluß erhitzt und nach dem Abkühlen auf Raumtemperatur mit 600 ml Wasser verdünnt. Die hierbei ausgefällten Kristalle wurden abgesaugt und mit Diethylether nachgewaschen. Auf diese Weise wurden 27,0 g 3-Phenyl-4-(acrylamidomethyl)-5-methyl-isoxazol erhalten, was einer Ausbeute von 79 Gew.% entspricht. Das Produkt hatte einen Schmelzpunkt von 149 bis 151°C.

| | | | | |
|---|---|---|---|---|
| Elementaranalyse: | C 69,4 | H 5,9 | O 13,1 | N 11,6 |
| Theorie: | C 69,41 | H 5,82 | O 13,21 | N 11,56 |

Die ¹H-NMR- und IR-spektroskopischen Messungen bestätigten das Vorliegen der gewünschten Verbindung.

### Beispiel 5

### 3-Phenyl-4-(acrylamidomethyl)-5-methyl-pyrazol

Eine Mischung aus 29,4 g des nach Beispiel 2 hergestellten 1-Phenyl-2-(acrylamidomethyl)-1,3-butandions, 15,6 g Hydraziniumhydrogensulfat, 19,7 g Natriumacetat, 180 ml Essigsäure und 180 ml Wasser wurde 5 Minuten lang am Rückfluß erhitzt und nach dem Abkühlen auf Raumtemperatur mit 600 ml Wasser verdünnt. Die hierbei ausgefällten Kristalle wurden abgesaugt und mit Diethylether nachgewaschen. Auf diese Weise wurden 23,1 g 3-Phenyl-4-(acrylamidomethyl)-5-methyl-pyrazol erhalten, entsprechend einer Ausbeute von 80 %. Das Produkt hatte einen Schmelzpunkt von 191 bis 193°C

| | | | |
|---|---|---|---|
| Elementaranalyse: | C 69,4 | H 6,3 | N 17,2 |
| Theorie: | C 69,69 | H 6,27 | N 17,41 |

Die ¹H-NMR- und IR-spektroskopischen Messungen bestätigten das Vorliegen der gewünschten Verbindung.

### Beispiel 6

### 1,3,5-Triphenyl-4-(acrylamidomethyl)-pyrazol

Ein Gemisch aus 67,4 g des nach Beispiel 3 hergestellten 1,3-Diphenyl-2-(acrylamidomethyl)-1,3-propandions, 23,8 g Phenylhydrazin, 230 ml Essigsäure und 50 ml Wasser wurde 6 Minuten auf 90°C erhitzt. Das nach dem Abkühlen auf Raumtemperatur auskristallisierte Produkt (58,2 g) wurde aus Ethanol umkristallisiert.

Das umkristallisierte Produkt wies einen Schmelzpunkt von 189 bis 191°C auf. Die ¹H-NMR-spektroskopische Messung bestätigte das Vorliegen von 1,3,5-Triphenyl-4-(acrylamidomethyl)-pyrazol:
¹H-NMR (Dimethylsulfoxid; 300 MHz):
δ = 4,20 (Doublett, 2H, -CH₂-)
δ = 5,60 (Doublett, 1H, H-C=C)
δ = 6,15 (Doublett, 1H, H-C=C)
δ = 6,30 (doppeltes Doublett, 1H, H-C=C)
δ = 7,2-7,8 (Multiplett, 15H, aromatische H)
δ = 8,60 (Triplett, 1H, NH)

### Beispiel 7

### 1,5-Diphenyl-3-methyl-4-(acrylamidomethyl)-pyrazol

Ein Gemisch aus 29,4 g des nach Beispiel 2 hergestellten 1-Phenyl-2-(acrylamidomethyl)-1,3-butandions, 12,9 g Phenylhydrazin, 180 ml Essigsäure und 180 ml Wasser wurde 5 Minuten am Rückfluß erhitzt und nach dem Abkühlen auf Raumtemperatur mit 600 ml Wasser verdünnt. Die hierbei ausgefällten Kristalle wurden abgesaugt und mit Diethylether nachgewaschen. Auf diese Weise wurden 37,4 g 1,5-Diphenyl-3-methyl-4-(acrylamidomethyl)-pyrazol mit einem Schmelzpunkt von 157 bis 159°C erhalten. Die ¹H-NMR-spektroskopische Messung bestätigte das Vorliegen dieses Produkts:
¹H-NMR (Dimethylsulfoxid; 300 MHz):
δ = 2,25 (Singulett, 3H, CH₃)
δ = 4,12 (Doublett, 2H, -CH₂-)
δ = 5,60 (Doublett, 1H, H-C=C)
δ = 6,12 (Doublett, 1H, H-C=C)
δ = 6,25 (doppeltes Doublett, 1H, H-C=C)
δ = 7,10-7,45 (Multiplett, 10H, aromatische H)
δ = 8,30 (Triplett, 1H, NH).

## Patentansprüche (Patentansprüche für folgende(n) Vertragsstaat(en): BE, DE, FR, GB, IT, NL, SE)

1. 2-[(Meth)Acrylamidomethyl]-1,3-diketone der allgemeinen Formel (I) 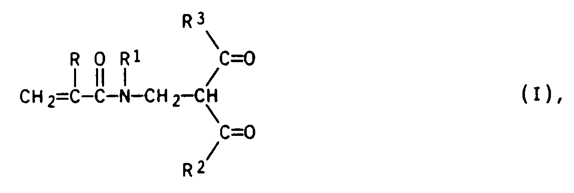 worin die Reste R, R¹, R² und R³, unabhängig voneinander, die folgende Bedeutung haben
R: ein Wasserstoffatom oder eine Methylgruppe;
R¹: ein Wasserstoffatom, eine Methyl- oder eine Ethyl-Gruppe;
R², R³: eine Alkylgruppe mit 1 bis 6 C-Atomen oder eine unsubstituierte oder substituierte Arylgruppe mit 6 bis 20 C-Atomen, wobei R² und R³ gleich oder voneinander verschieden sein können.

2. 2-[(Meth)Acrylamidomethyl]-1,3-diketone nach Anspruch 1, dadurch gekennzeichnet, daß in der allgemeinen Formel (I) R und R¹ jeweils ein Wasserstoffatom sowie R² und R³ eine Methyl- und/oder Phenylgruppe bedeuten.

3. Verfahren zur Herstellung von 2-[(Meth)Acrylamidomethyl]-1,3-diketonen der allgemeinen Formel (I) gemäß einem der Ansprüche 1 oder 2, dadurch gekennzeichnet, daß man
(i) ein 1,3-Diketon der allgemeinen Formel (II) 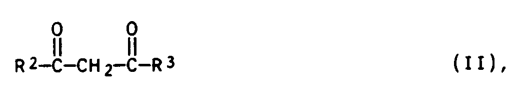 worin R² und R³ gleich oder voneinander verschieden sein können und eine Alkylgruppe mit 1 bis 6 C-Atomen oder eine unsubstituierte oder substituierte Arylgruppe mit 6 bis 20 C-Atomen bedeuten, mit
(ii) einem N-Methylol-(meth)acrylamid der allgemeinen Formel (III) 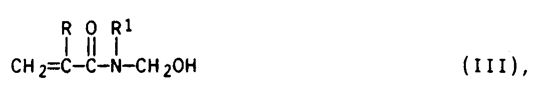 worin R ein Wasserstoffatom oder eine Methylgruppe und R¹ ein Wasserstoffatom, eine Methyl- oder eine Ethylgruppe bedeuten,
(iii) in einer starken Säure als Reaktionsmedium kondensiert.

4. Verfahren nach Anspruch 3, dadurch gekennzeichnet, daß in der allgemeinen Formel (III) die Reste R und R¹ jeweils ein Wasserstoffatom und daß in der allgemeinen Formel (II) die Reste R² und R³ eine Methyl- und/oder Phenyl-Gruppe bedeuten.

5. Verfahren nach Anspruch 3 oder 4, dadurch gekennzeichnet, daß die als Reaktionsmedium verwendete starke Säure Schwefelsäure, Trifluormethansulfonsäure oder Phosphorsäure ist.

6. Verfahren nach Anspruch 5, dadurch gekennzeichnet, daß man als Reaktionsmedium konzentrierte Schwefelsäure verwendet.

7. Verfahren nach einem der Ansprüche 3 bis 6, dadurch gekennzeichnet, daß man die Kondensationsreaktion in der Gegenwart eines thermischen Polymerisationsinhibitors durchführt.

8. Verwendung der 2-[(Meth)Acrylamidomethyl]-1,3-diketone gemäß Anspruch 1 oder 2 zur Herstellung polymerisierbarer heterocyclischer Verbindungen.

9. Verwendung der 2-[(Meth)Acrylamidomethyl]-1,3-diketone gemäß Anspruch 1 oder 2 zur Herstellung polymerisierbarer Metallchelat-Komplexe.

10. Verwendung der 2-[(Meth)Acrylamidomethyl]-1,3-diketone gemäß Anspruch 1 oder 2 für die Herstellung von Polymerisaten, welche zur Metallchelat-Komplexbildung befähigt sind.

11. Verwendung der 2-[(Meth)Acrylamidomethyl]-1,3-diketone gemäß Anspruch 1 oder 2 als photopolymerisierbare Monomere in lichtempfindlichen Aufzeichnungsmaterialien.

## Patentansprüche (Patentansprüche für folgende(n) Vertragsstaat(en): ES)

1. Verfahren zur Herstellung von 2-[(Meth)Acrylamidomethyl]-1,3-diketonen der allgemeinen Formel (I) 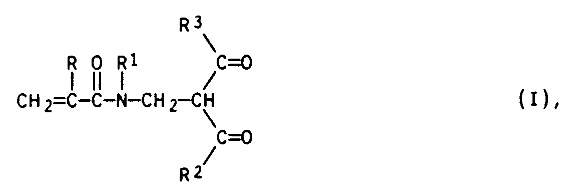 worin die Reste R, R¹, R² und R³, unabhängig voneinander, die folgende Bedeutung haben
R: ein Wasserstoffatom oder eine Methylgruppe;
R¹: ein Wasserstoffatom, eine Methyl- oder eine Ethylgruppe;
R², R³: eine Alkylgruppe mit 1 bis 6 C-Atomen oder eine unsubstituierte oder substituierte Arylgruppe mit 6 bis 20 C-Atomen, wobei R² und R³ gleich oder voneinander verschieden sein können;
dadurch gekennzeichnet, daß man
(i) ein 1,3-Diketon der allgemeinen Formel (II) 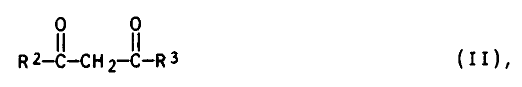 worin R² und R³ gleich oder voneinander verschieden sein können und eine Alkylgruppe mit 1 bis 6 C-Atomen oder eine unsubstituierte oder substituierte Arylgruppe mit 6 bis 20 C-Atomen bedeuten, mit
(ii) einem N-Methylol-(meth)acrylamid der allgemeinen Formel (III) 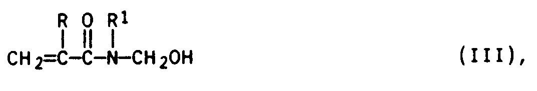 worin R ein Wasserstoffatom oder eine Methylgruppe und R¹ ein Wasserstoffatom, eine Methyl- oder eine Ethylgruppe bedeuten,
(iii) in einer starken Säure als Reaktionsmedium kondensiert.

2. Verfahren nach Anspruch 1, dadurch gekennzeichnet, daß in den allgemeinen Formeln (I) und (III) die Reste R und R¹ jeweils ein Wasserstoffatom und daß in den allgemeinen Formeln (I) und (II) die Reste R² und R³ eine Methyl- und/oder Phenylgruppe bedeuten.

3. Verfahren nach Anspruch 1 oder 2, dadurch gekennzeichnet, daß die als Reaktionsmedium verwendete starke Säure Schwefelsäure, Trifluormethansulfonsäure oder Phosphorsäure ist.

4. Verfahren nach Anspruch 3, dadurch gekennzeichnet, daß man als Reaktionsmedium konzentrierte Schwefelsäure verwendet.

5. Verfahren nach einem der Ansprüche 1 bis 5, dadurch gekennzeichnet, daß man die Kondensationsreaktion in der Gegenwart eines thermischen Polymerisationsinhibitors durchführt.

6. Verwendung der gemäß einem der Ansprüche 1 bis 5 hergestellten 2-[(Meth)Acrylamidomethyl]-1,3-diketone zur Herstellung polymerisierbarer heterocyclischer Verbindungen.

7. Verwendung der gemäß einem der Ansprüche 1 bis 5 hergestellten 2-[(Meth)Acrylamidomethyl]-1,3-diketone zur Herstellung polymerisierbarer Metallchelat-Komplexe.

8. Verwendung der gemäß einem der Ansprüche 1 bis 5 hergestellten 2-[(Meth)Acrylamidomethyl]-1,3-diketone für die Herstellung von Polymerisaten, welche zur Metallchelat-Komplexbildung befähigt sind.

9. Verwendung der gemäß einem der Ansprüche 1 bis 5 hergestellten 2-[(Meth)Acrylamidomethyl]-1,3-diketone als photopolymerisierbare Monomere in lichtempfindlichen Aufzeichnungsmaterialien.

## Claims (Claims for the following Contracting State(s): BE, DE, FR, GB, IT, NL, SE)

1. A 2-[(meth)acrylamidomethyl]-1,3-diketone of the general formula (I) 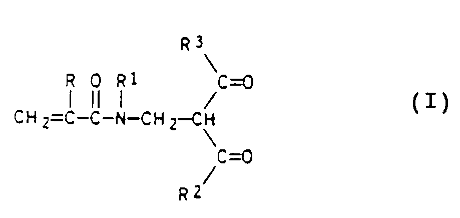 where R, R¹, R² and R³ are each independently of the others defined as follows:
R as hydrogen or methyl,
R¹ as hydrogen, methyl or ethyl,
R² and R³ each as alkyl of from 1 to 6 carbon atoms or unsubstituted or substituted aryl of from 6 to 20 carbon atoms, R² and R³ being identical to or different from each other.

2. A 2-[(meth)acrylamidomethyl]-1,3-diketone as claimed in claim 1, wherein in the general formula (I) R and R¹ are each hydrogen and R² and R³ are each methyl or phenyl.

3. A process for preparing a 2-[(meth)acrylamido-methyl]-1,3-diketone of the general formula (I) as claimed in claim 1 or 2, which comprises condensing
(i) a 1,3-diketone of the general formula (II) 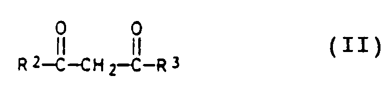 where R² and R³ are identical to or different from each other and each is alkyl of from 1 to 6 carbon atoms or unsubstituted or substituted aryl of from 6 to 20 carbon atoms, with
(ii) an N-methylol(meth)acrylamide of the general formula (III) 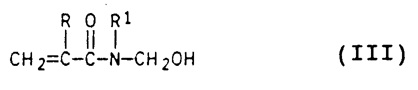 where R is hydrogen or methyl and R¹ is hydrogen, methyl or ethyl,
(iii) in a strong acid as the reaction medium.

4. A process as claimed in claim 3, wherein in the general formula (III) R and R¹ are each hydrogen and in the general formula (II) R² and R³ are each methyl or phenyl.

5. A process as claimed in claim 3 or 4, wherein the strong acid used as the reaction medium is sulfuric acid, trifluoromethanesulfonic acid or phosphoric acid.

6. A process as claimed in claim 5, wherein the reaction medium used is concentrated sulfuric acid.

7. A process as claimed in any of claims 3 to 6, wherein the condensation reaction is carried out in the presence of a thermal polymerization inhibitor.

8. The use of 2-[(meth)acrylamidomethyl]-1,3-diketones as claimed in claim 1 or 2 for preparing polymerizable heterocyclic compounds.

9. The use of 2-[(meth)acrylamidomethyl]-1,3-diketones as claimed in claim 1 or 2 for preparing polymerizable metal chelates.

10. The use of 2-[(meth)acrylamidomethyl]-1,3-diketones as claimed in claim 1 or 2 for preparing polymers capable of metal chelation.

11. The use of 2-[(meth)acrylamidomethyl]-1,3-diketones as claimed in claim 1 or 2 as photopolymerizable monomers in light-sensitive recording materials.

## Claims (Claims for the following Contracting State(s): ES)

1. A process for preparing a 2-[(meth)acrylamidomethyl]-1,3-diketone of the general formula (I) 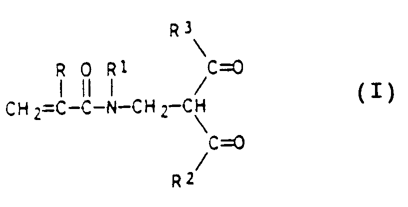 where R, R¹, R² and R³ are each independently of the others defined as follows:
R as hydrogen or methyl,
R¹ as hydrogen, methyl or ethyl,
R² and R³ each as alkyl of from 1 to 6 carbon atoms or unsubstituted or substituted aryl of from 6 to 20 carbon atoms, R² and R³ being identical to or different from each other,
which comprises condensing
(i) a 1,3-diketone of the general formula (II) 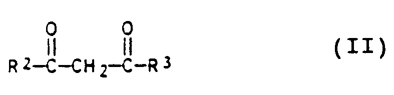 where R² and R³ are identical to or different from each other and each is alkyl of from 1 to 6 carbon atoms or unsubstituted or substituted aryl of from 6 to 20 carbon atoms, with
(ii) an N-methylol(meth)acrylamide of the general formula (III) 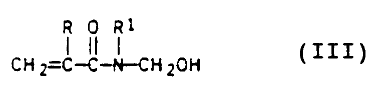 where R is hydrogen or methyl and R¹ is hydrogen, methyl or ethyl,
(iii) in a strong acid as the reaction medium.

2. A process as claimed in claim 1, wherein in the general formulae (I) and (III) R and R¹ are each hydrogen and in the general formulae (I) and (II) R² and R³ are each methyl or phenyl.

3. A process as claimed in claim 1 or 2, wherein the strong acid used as the reaction medium is sulfuric acid, trifluoromethanesulfonic acid or phosphoric acid.

4. A process as claimed in claim 3, wherein the reaction medium used in concentrated sulfuric acid.

5. A process as claimed in any of claims 1 to 5, wherein the condensation reaction is carried out in the presence of a thermal polymerization inhibitor.

6. The use of the 2-[(meth)acrylamidomethyl]-1,3-diketones prepared as claimed in any one of claims 1 to 5 for preparing polymerizable heterocyclic compounds.

7. The use of the 2-[(meth)acrylamidomethyl]-1,3-diketones prepared as claimed in any one of claims 1 to 5 for preparing polymerizable metal chelates.

8. The use of the 2-[(meth)acrylamidomethyl]-1,3-diketones prepared as claimed in any one of claims 1 to 5 for preparing polymers capable of metal chelation.

9. The use of the 2-[(meth)acrylamidomethyl]-1,3-diketones prepared as claimed in any one of claims 1 to 5 as photopolymerizable monomers in light-sensitive recording materials.

## Revendications (Revendications pour l'(les) Etat(s) contractant(s) suivant(s): BE, DE, FR, GB, IT, NL, SE)

1. 2-[(Méth)acrylamidométhyl]-1,3-dicétones de la formule générale (I) 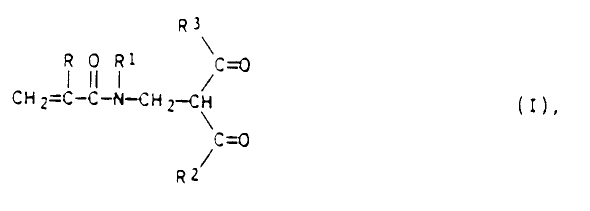 dans laquelle les symboles R, R¹, R² et R³ ont, indépendamment les uns des autres, les significations suivantes :
R: atome d'hydrogène ou radical méthyle,
R¹: atome d'hydrogène, radical méthyle ou éthyle,
R², R³: radical alkyle comportant de 1 à 6 atomes de carbone, ou radical aryle non substitué ou substitué, qui comporte de 6 à 20 atomes de carbone, où R² et R³ peuvent être identiques ou différer mutuellement.

2. 2-[(Méth)acrylamidométhyl]-1,3-dicétones selon la revendication 1, caractérisées en ce que, dans la formule générale (I), R et R¹ représentent chacun un atome d'hydrogène, tout comme R² et R³ représentent un radical méthyle et/ou phényle.

3. Procédé de préparation de 2-[(méth)acrylamidométhyl]-1,3-dicétones de la formule générale (I) selon l'une des revendications 1 et 2, caractérisé en ce que l'on condense
(i) une 1,3-dicétone de la formule (II) 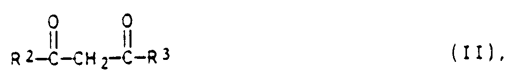 dans laquelle R² et R³ sont identiques ou diffèrent mutuellement et représentent chacun un radical alkyle comportant de 1 à 6 atomes de carbone, ou un radical aryle non substitué ou substitué, comportant de 6 à 20 atomes de carbone, avec
(ii) un N-méthylol-(méth)acrylamide de la formule générale (III) 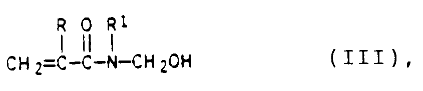 dans laquelle R représente un atome d'hydrogène ou le radical méthyle et R¹ représente un atome d'hydrogène, le radical méthyle ou éthyle,
(iii) dans un acide fort servant de milieu de réaction.

4. Procédé suivant la revendication 3, caractérisé en ce que, dans la formule générale (II), les symboles R et R¹ représentent chacun un atome d'hydrogène et en ce que, dans la formule générale (II), les symboles R² et R³ représentent un radical méthyle et/ou phényle.

5. Procédé suivant la revendication 3 ou 4, caractérisé en ce que l'acide fort utilisé à titre de milieu de réaction est l'acide sulfurique, l'acide trifluorométhanesulfonique ou l'acide phosphorique.

6. Procédé suivant la revendication 5, caractérisé en ce que l'on utilise de l'acide sulfurique concentré à titre de milieu de réaction.

7. Procédé selon l'une quelconque des revendications 3 à 6, caractérisé en ce que l'on entreprend la réaction de condensation en présence d'un inhibiteur de polymérisation thermique.

8. Utilisation des 2-[(méth)acrylamidométhyl]-1,3-dicétones selon la revendication 1 ou 2 en vue de la préparation de composés hétérocycliques polymérisables.

9. Utilisation des 2-[(méth)acrylamidométhyl]-1,3-dicétones selon la revendication 1 ou 2 en vue de la préparation de complexes de chélates de métaux polymérisables.

10. Utilisation des 2-[(méth)acrylamidométhyl]-1,3-dicétones selon la revendication 1 ou 2 en vue de la préparation de polymères qui sont susceptibles de former des complexes de chélates de métaux.

11. Utilisation des 2-[(méth)acrylamidométhyl]-1,3-dicétones selon la revendication 1 ou 2 à titre de monomères photopolymérisables dans des matériaux d'enregistrement photosensibles.

## Revendications (Revendications pour l'(les) Etat(s) contractant(s) suivant(s): ES)

1. Procédé de préparation de 2-[méth)acrylamidométhyl]-1,3-dicétones de la formule générale (I) 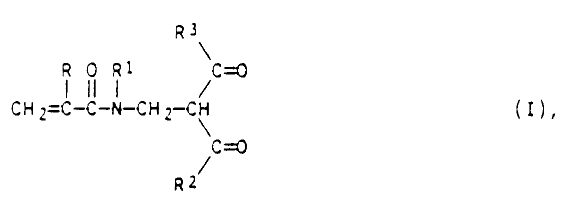 dans laquelle les symboles R, R¹, R² et R³ ont, indépendamment les uns des autres, les significations suivantes :
R: atome d'hydrogène ou radical méthyle,
R¹: atome d'hydrogène, radical méthyle ou éthyle,
R², R³: radical alkyle comportant de 1 à 6 atomes de carbone, ou radical aryle non substitué ou substitué, qui comporte de 6 à 20 atomes de carbone, où R² et R³ peuvent être identiques ou différer mutuellement;
caactérisé en ce que l'on condense
(i) une 1,3-dicétone de la formule (II) 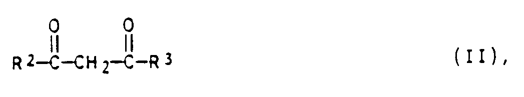 dans laquelle R² et R³ sont identiques ou diffèrent mutuellement et représentent chacun un radical alkyle comportant de 1 à 6 atomes de carbone, ou un radical aryle non substitué ou substitué, comportant de 6 à 20 atomes de carbone, avec
(ii) un N-méthylol(méth)acrylamide de la formule générale (III) 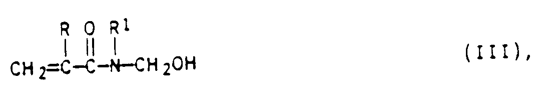 dans laquelle R représente un atome d'hydrogène ou le radical méthyle et R¹ représente un atome d'hydrogène, le radical méthyle ou éthyle,
(iii) dans un acide fort servant de milieu de réaction.

2. Procédé suivant la revendication 1, caractérisé en ce que, dans les formules générales (I) et (III), les symboles R et R¹ représentent chacun un atome d'hydrogène et en ce que, dans les formules générales (I) et (II), les symboles R² et R³ représentent chacun un groupe méthyle et/ou phényle.

3. Procédé selon la revendication 1 ou 2, caractérisé en ce que l'acide fort utilisé à titre de milieu de réaction est l'acide sulfurique, l'acide trifluorométhanesulfonique ou l'acide phosphorique.

4. Procédé selon la revendication 3, caractérisé en ce que l'on utilise l'acide sulfurique concentré à titre de milieu de réaction.

5. Procédé selon l'une quelconque des revendications 1 à 5, caractérisé en ce que l'on entreprend la réaction de condensation en présence d'un inhibiteur de polymérisation thermique.

6. Utilisation des 2-[(méth)acrylamidométhyl]-1,3-dicétones préparées selon l'une quelconque des revendications 1 à 5, en vue de la préparation de composés hétérocycliques polymérisables.

7. Utilisation des 2-[méth)acrylamidométhyl]-1,3-dicétones préparées selon l'une quelconque des revendications 1 à 5, en vue de la préparation de complexes de chélates de métaux polymérisables.

8. Utilisation des 2-[(méth)acrylamidométhyl]-1,3-dicétones préparées selon l'une quelconque des revendications 1 à 5, en vue de la préparation d epolymères qui sont susceptibles de former des complexes avec des chélates de métaux.

9. Utilisation des 2-[(méth)acrylamidométhyl]-1,3-dicétones préparées selon l'une quelconque des revendications 1 à 5, à titre de monomères photopolymérisables dans des matériaux d'enregistrement photosensibles.
